# EUROPEAN PATENT SPECIFICATION

(11) **EP 3 976 144 B1**
(45) Date of publication and mention of the grant of the patent: **12.02.2025**
(21) Application number: 20727295.6
(22) Date of filing: 22.05.2020
(51) Int. Cl.: A61M 11/04, A24F 40/42, A24F 40/485, A61M 15/06

(54) **AEROSOL DELIVERY DEVICE**
AEROSOLABGABEVORRICHTUNG
DISPOSITIF DE DISTRIBUTION D'AÉROSOL

(30) Priority: 24.05.2019 EP 19176370; 24.05.2019 EP 19176406; 24.05.2019 EP 19176400; 24.05.2019 EP 19176386; 24.05.2019 EP 19176420; 24.05.2019 EP 19176408; 24.05.2019 EP 19176398; 24.05.2019 EP 19176391
(43) Date of publication of application: 06.04.2022
(60) Divisional application: 24219933.9
(73) Proprietor: Imperial Tobacco Limited, Bristol, BS3 2LL (GB)
(72) Inventor: AUSTIN, Andrew, Liverpool Merseyside L24 9HP (GB); SAJTOS, Tamas, Liverpool Merseyside L24 9HP (GB); COOK, Alan, Liverpool Merseyside L24 9HP (GB); LOMAS, Pete, Liverpool Merseyside L24 9HP (GB); TYLER, Andrew, Liverpool Merseyside L24 9HP (GB)
(74) Representative: Mewburn Ellis LLP
(86) International application number: PCT/EP2020/064320
(87) International publication number: WO 2020/239636

(56) References cited:
- EP-A1- 3 011 850
- WO-A1-2017/167511
- GB-A- 2 566 774
- US-A1- 2015 027 470
- US-A1- 2017 013 880
- US-A1- 2017 071 249
- US-A1- 2017 196 268

## Description

### Field of the Disclosure

The present disclosure relates to an aerosol delivery device and particularly, although not exclusively, to an aerosol delivery device having a barrier arrangement e.g. to inhibit evaporation of aerosol precursor. The present disclosure also relates to an aerosol delivery device comprising a frangible seal.

### Background

A smoking-substitute device is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heating element. The consumable may also be referred to as a cartomizer. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu(RTM) e-cigarette. The myblu(RTM) e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

Patent document US 2017/071249 A1 proposes a reservoir for aerosol delivery devices. Patent document US 2017/196268 A1 proposes an element for an aerosol-generating system comprising a disabling mechanism. WO2017167511 proposes an aerosol generating system comprising a capsule and a vaporizing unit releasably connectable to the capsule. The capsule comprises a reservoir for containing a liquid aerosol generating substrate, an opening in fluid communication with the reservoir, and a valve configured to control flow of the liquid aerosol-generating substrate from the reservoir through the opening. The valve comprises one or more resilient closing members biased towards a closed position.

GB 2566774A proposes an aerosol delivery device comprising a passive aerosol generator In aerosol delivery devices with passive aerosol generation, it is desirable to prevent evaporation of aerosol precursor when the device is not being used.

In aerosol delivery devices, it is desirable to prolong the storage time of such devices without impacting on the quantity or quality of the product.

The present disclosure has been devised in light of the above considerations.

### Summary

The invention is defined by the appended claims. Subject-matter referred as embodiments, aspects and/or disclosures which do not fall under the scope of the claims are not part of the invention. If

Generally this application relates to an aerosol delivery device in which a barrier arrangement inhibits evaporation of aerosol precursor when the barrier arrangement in closed, and the barrier arrangement is openable to permit generation of aerosol.

In a first aspect, there is provided aerosol delivery device according to claim 1.

Such an aerosol delivery device is less prone to leakage during transit. Further, evaporation of the aerosol precursor can be prevented when the device is not in use.

Optionally, the barrier arrangement may be configured to open in response to a user inhaling on the aerosol delivery device.

Advantageously, the barrier arrangement may be selectively openable by a user.

Conveniently, the barrier arrangement may further comprise a mouthpiece and a body, wherein the barrier arrangement is configured to open in response to sliding of the mouthpiece relative to the body.

The barrier arrangement comprises a barrier element between the aerosol generator portion and a mouthpiece aperture of the aerosol delivery device to inhibit evaporation of aerosol precursor.

Advantageously, the barrier element may comprise a non-return valve.

Conveniently, the barrier element may comprise a movable shield.

Optionally, the barrier arrangement may be configured to inhibit flow of aerosol precursor between the storage and the aerosol generator portion to inhibit evaporation of aerosol precursor.

Advantageously, the aerosol delivery device may further comprise a transfer element, wherein the transfer element is movable with respect to the barrier arrangement to open the barrier arrangement so that the transfer element can transfer aerosol precursor from the storage to the aerosol generator portion.

Conveniently, the aerosol delivery device may further comprise a member, the member comprising the transfer element and the aerosol generator portion, wherein the member is movable from a first position to a second position to open the barrier arrangement.

Optionally, the aerosol delivery device may further comprise a supporting portion for maintaining a position of the member with respect to the mouthpiece during sliding of the mouthpiece.

Advantageously, the aerosol delivery device may further comprise: a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

In a second aspect, there is provided an aerosol delivery device comprising: a storage for storing an aerosol precursor; a transfer element; and a barrier arrangement for inhibiting flow of aerosol precursor from the storage to the transfer element when the barrier arrangement is in a closed position, wherein the barrier arrangement is selectively actuatable between the closed position and an open position, and when the barrier arrangement is in the open position the transfer element can transfer aerosol precursor from the storage.

Such an aerosol delivery device can be used to selectively provide aerosol to the user.

The barrier arrangement may be referred to as a charging or filling mechanism, operable to charge, recharge, fill, or refill the transfer element with aerosol precursor. The transfer element may be for transferring aerosol precursor to an aerosol generator of the aerosol delivery device.

Optionally, the transfer element may be selectively exposable to the storage to selectively open and close the barrier arrangement.

Advantageously, the transfer element may be movable with respect to the barrier arrangement to selectively expose the transfer element to the storage.

Conveniently, the barrier arrangement may comprise a barrier element configured to move with the transfer element, the barrier element configured to inhibit flow of aerosol precursor from the storage to the transfer element when the barrier arrangement is in the closed position.

Optionally, the barrier arrangement may further comprise a tube, the transfer element received in the tube and the barrier element configured to block the tube when the barrier arrangement is in the closed position.

Advantageously, the barrier element may be fixed to an end portion of the transfer element, and the barrier element may be configured to be out of the tube such that a side portion of the transfer element is exposed to the storage when the barrier arrangement is in the open position.

Conveniently, the aerosol delivery device may further comprise an aerosol generator configured to receive aerosol precursor from the storage and form an aerosol from the aerosol precursor.

Optionally, the aerosol generator may comprise an aerosol generator portion configured to receive the aerosol precursor from the storage, and the aerosol delivery device comprises an air flow passage configured to direct air past the aerosol generator portion to pick up the aerosol precursor from the aerosol generator portion to form an aerosol.

Advantageously, the aerosol delivery device may further comprise a member, the member comprising the transfer element and the aerosol generator portion.

Conveniently, the member may be movable with respect to the barrier arrangement to selectively expose the transfer element to the storage.

Optionally, the aerosol delivery device may further comprise a mouthpiece and a body, wherein the barrier arrangement is configured to open in response to sliding of the mouthpiece relative to the body.

Advantageously, the transfer element may be porous.

In a third aspect, there is provided an aerosol delivery device comprising: a storage for storing an aerosol precursor; an additional storage; and a barrier arrangement for inhibiting flow of aerosol precursor from the storage to the additional storage, the barrier arrangement openable to permit flow of aerosol precursor from the storage to the additional storage.

Such an aerosol delivery device is less prone to leakage during transit.

Optionally, the aerosol delivery device may comprise an aerosol generator configured to receive aerosol precursor from the additional storage and form an aerosol from the aerosol precursor.

Advantageously, the aerosol generator may comprise an aerosol generator portion configured to receive the aerosol precursor from the additional storage, and the aerosol delivery device may comprise an air flow passage configured to direct air past the aerosol generator portion to pick up the aerosol precursor from the aerosol generator portion to form an aerosol.

Conveniently, the aerosol delivery device may comprise a transfer element for transferring aerosol precursor from the additional storage to the aerosol generator portion. The transfer element may be at least partially contained within the additional storage. The additional storage is, in some examples, a container into which aerosol precursor can freely flow once the barrier mechanism is opened.

Optionally, the aerosol delivery device may comprise a member, the member comprising the transfer element and the aerosol generator portion.

Advantageously, the transfer element may be porous.

Conveniently, the barrier arrangement may comprise a barrier component between the storage and the additional storage, and the transfer element is movable to pierce the barrier component to open the barrier arrangement.

Optionally, the barrier component may be a foil membrane.

Advantageously, the aerosol delivery device may comprise a mouthpiece and a body, wherein the barrier arrangement is configured to open in response to sliding of the mouthpiece relative to the body.

Conveniently, the aerosol delivery device may comprise a supporting portion for maintaining a position of the transfer element with respect to the mouthpiece during sliding of the mouthpiece.

Optionally, the storage may comprise a free liquid tank and the additional storage comprises a porous reservoir.

Advantageously, the additional storage may be located closer to a mouthpiece opening than the storage.

In a fourth aspect, there is provided aerosol delivery device comprising: a storage for storing an aerosol precursor; a transfer element; and a barrier arrangement comprising a plug, the plug configured to inhibit flow of aerosol precursor from the storage to the transfer element when the plug is in a closed position, wherein the plug is displaceable to open the barrier arrangement so that the transfer element can transfer aerosol precursor from the storage.

Such an aerosol delivery device is less prone to leakage during transit. The transfer element may be for transferring aerosol precursor from the storage to an aerosol generator of the aerosol delivery device.

Optionally, the transfer element may be movable to displace the plug to open the transfer element.

Advantageously, the aerosol delivery device may comprise a tube, wherein the transfer element and the plug are received in the tube, and the plug is displaceable from the tube to open the barrier arrangement.

Conveniently, the aerosol delivery device may comprise an aerosol generator comprising an aerosol generator portion configured to receive the aerosol precursor from the transfer element; and an air flow passage configured to direct air past the aerosol generator portion to pick up the aerosol precursor from the aerosol generator portion to form an aerosol.

Optionally, the aerosol delivery device may comprise a member, the member comprising the transfer element and the aerosol generator portion, wherein the member is movable from a first position to a second position to open the barrier arrangement.

Advantageously, the aerosol delivery device may comprise a guide for inhibiting return of the plug to the closed position after displacement of the plug.

Conveniently, the guide may comprise a recess for receiving the plug.

Optionally, the plug may be formed of silicone.

Advantageously, the aerosol delivery device may comprise a mouthpiece and a body, wherein the barrier arrangement is configured to open in response to sliding of the mouthpiece relative to the body.

Conveniently, the aerosol delivery device may comprise a supporting portion for maintaining a position of the member with respect to the mouthpiece during sliding of the mouthpiece.

Optionally, the aerosol delivery device may comprise a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

Advantageously, the transfer element may be porous.

In a fifth aspect, there is provided an aerosol delivery device comprising: a storage for storing an aerosol precursor; a transfer element; and a barrier arrangement for inhibiting flow of aerosol precursor from the storage to the transfer element, wherein the storage is movable with respect to the barrier arrangement to open the barrier arrangement so that the transfer element can transfer aerosol precursor from the storage.

Such an aerosol delivery device is less prone to leakage during transit. The transfer element may be for transferring aerosol precursor from the storage to an aerosol generator of the aerosol delivery device. The storage maybe movable relative to a mouthpiece of the aerosol delivery device, and this movement may cause the barrier arrangement to be opened. The barrier arrangement may be fixed relative to the mouthpiece until the barrier arrangement is opened.

Optionally, the storage may be movable relative to the transfer element to open the barrier arrangement.

Advantageously, the barrier arrangement may comprise a plug received in a tube, the plug configured to inhibit flow of aerosol precursor from the storage to the transfer element, wherein the tube is configured to move with the storage, and the plug is displaceable from the tube on movement of the storage so that the transfer element can transfer aerosol precursor from the storage.

Conveniently, movement of the storage may cause the transfer element to contact the plug to displace the plug from the tube. For example, the plug may be freely placed in the tube (or held only by a relatively light interference fit). Thus, when the tube is moved relative to the transfer element, the plug will no longer be retained within the tube.

Optionally, the barrier arrangement may comprise a deformable barrier component, the barrier component configured to deform to open the barrier arrangement on movement of the storage.

Advantageously, movement of the storage may cause the transfer element to pierce the barrier component to open the barrier arrangement.

Conveniently, the aerosol delivery device may comprise a slidable switch for moving the storage.

Optionally, the aerosol delivery device may comprise a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

Advantageously, the moving the switch may cause the blocking arrangement to open.

Conveniently, the connector may provide the blocking arrangement, and the aerosol delivery device further comprises a stop, the stop configured to inhibit movement of the storage after the barrier arrangement is open, such that further movement of the switch causes the connector to disconnect from the storage to open the blocking arrangement.

Optionally, the aerosol delivery device may further comprise an aerosol generator comprising an aerosol generator portion configured to receive the aerosol precursor from the transfer element; and an air flow passage configured to direct air past the aerosol generator portion to pick up the aerosol precursor from the aerosol generator portion to form an aerosol.

Advantageously, the aerosol delivery device may further comprise a member, the member comprising the transfer element and the aerosol generator portion, wherein the member is movable from a first position to a second position to open the barrier arrangement.

Conveniently, the aerosol delivery device may further comprise a mouthpiece and a supporting portion for maintaining a position of the member with respect to the mouthpiece during movement of the storage.

In a sixth aspect, there is provided aerosol delivery device comprising: a storage for storing an aerosol precursor; a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

Optionally, the aerosol delivery device may comprise a mouthpiece and a body, the mouthpiece movable relative to the body to open the blocking arrangement.

Advantageously, twisting of the mouthpiece relative to the body may cause opening of the blocking arrangement.

Conveniently, sliding of the mouthpiece relative to body may cause opening of the blocking arrangement.

Optionally, the blocking arrangement may comprise a blocking component for inhibiting flow through the pressure relief opening, the blocking component defining an aperture, the aperture alignable with the pressure relief opening to open the blocking arrangement.

Advantageously, the blocking arrangement may comprise a blocking component for inhibiting flow through the pressure relief opening and a piercing component movable relative to the blocking component, wherein the blocking component is pierceable by the piercing component to open the blocking arrangement.

Conveniently, the aerosol delivery device may comprise a transfer element; and a barrier arrangement for inhibiting flow of aerosol precursor from the storage to the transfer element, the barrier arrangement openable to permit flow of aerosol precursor from the storage to the transfer element.

Optionally, the aerosol delivery device may comprise an aerosol generator comprising an aerosol generator portion configured to receive the aerosol precursor from the transfer element; and an air flow passage configured to direct air past the aerosol generator portion to pick up the aerosol precursor from the aerosol generator portion to form an aerosol.

Advantageously, the aerosol delivery device may comprise a member, the member comprising the transfer element and the aerosol generator portion, wherein the member is movable from a first position to a second position to open the barrier arrangement.

Conveniently, the aerosol delivery device may comprise a supporting portion for maintaining a position of the member with respect to the mouthpiece during sliding of the mouthpiece.

Optionally, opening of the barrier arrangement may cause open of the blocking arrangement.

Advantageously, the member may be slidable from the first position to the second position to open the barrier arrangement.

In a seventh aspect, there is provided aerosol delivery device comprising: a storage for storing an aerosol precursor; a transfer element; and a barrier arrangement for inhibiting flow of aerosol precursor from the storage to the transfer element, wherein the transfer element is movable with respect to the barrier arrangement to open the barrier arrangement so that the transfer element can transfer aerosol precursor from the storage.

Optionally, the aerosol delivery device may further comprise an aerosol generator comprising an aerosol generator portion configured to receive the aerosol precursor from the transfer element; and an air flow passage configured to direct air past the aerosol generator portion to pick up the aerosol precursor from the aerosol generator portion to form an aerosol. The transfer element may be configured to transfer aerosol precursor from the storage to the aerosol generator once the barrier arrangement is open.

Advantageously, the aerosol generator portion may further comprise a member, the member comprising the transfer element and the aerosol generator portion, wherein the member is movable from a first position to a second position to open the barrier arrangement.

Conveniently, the member may be slidable from the first position to the second position to open the barrier arrangement.

Optionally, the aerosol delivery device may further comprise a mouthpiece and a body, wherein the barrier arrangement is configured to open in response to sliding of the mouthpiece relative to the body.

Advantageously, the aerosol delivery device may further comprise a supporting portion for maintaining a position of the member with respect to the mouthpiece during sliding of the mouthpiece.

Conveniently, the barrier arrangement may be configured to remain permanently open after opening.

Optionally, the barrier arrangement may comprise a plug received in a tube, the plug configured to inhibit flow of aerosol precursor from the storage to the transfer element, wherein the plug is displaceable from the tube on movement of the transfer element so that the transfer element can transfer aerosol precursor from the storage.

Advantageously, the aerosol delivery device may further comprise a guide for inhibiting return of the plug to the tube after the plug is displaced from the tube.

Conveniently, the barrier arrangement may comprise a deformable barrier component, and the transfer element is configured to deform the barrier component to open the barrier arrangement.

Optionally, the aerosol delivery device may further comprise a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

Advantageously, the transfer element may be porous.

The present disclosure also relates to an aerosol delivery device comprising a frangible seal between a reservoir and an aerosol generator.

In an eighth aspect, there is provided aerosol delivery device comprising:
a reservoir containing an aerosol precursor;
an aerosol generator, operable to aerosolise the aerosol precursor and provide it to a user; and
a frangible seal, which is located between the reservoir and the aerosol generator, and which, when unbroken, fluidly seals the aerosol generator from the reservoir and which, when broken, allows the aerosol precursor to fluidly contact the aerosol generator.

Such an aerosol delivery device may be stored for a longer period of time without degradation of the aerosol precursor. Further, such an aerosol delivery device is less prone to leakage during transportation.

Optionally, the device may further comprise an aerosol generator portion configured to receive the aerosol precursor, when the frangible seal is broken, and an airflow passage, configured to direct air past the aerosol generator portion to pick up the aerosol precursor from the aerosol generator portion to form an aerosol.

Advantageously, the aerosol generator portion may include a Venturi aperture and a porous member located within the Venturi aperture and fluidly connectable to the reservoir.

Conveniently, the device may further comprise a housing of the reservoir which is configured to deform and break the frangible seal. Optionally, the device may further comprise an outer housing, adjacent to the housing of the reservoir and configured to deform upon application of a force and impact the housing of the reservoir.

Advantageously, the frangible seal may be a cylindrical glass seal encapsulating a region of the aerosol generator. Conveniently, the aerosol generator may comprise a nib and a shaft, and the shaft may be disposed within the cylindrical glass seal.

Conveniently, the frangible seal may be configured to break when a force of greater than 10 kgf is applied.

Optionally, the device may further comprise a button which, when pressed, breaks the frangible seal.

Advantageously, the device may further comprise a vapour generator, for vaporising a vapour precursor, and the aerosol generator by the aerosol generator may be mixed with the vapour downstream of the vapour generator.

Conveniently, the aerosol precursor may be a flavour aerosol precursor and may be substantially nicotine free.

Optionally, the aerosol generator may be made of a material having a different colour to the aerosol precursor.

Advantageously, the aerosol delivery device may have a longitudinal axis, and the frangible seal may be configured to break upon application of a force transversal to the longitudinal axis.

Conveniently, the aerosol delivery device is a consumable for a smoking substitute device.

Optionally, the device further comprises an additional aerosol generator, the additional aerosol generator being configured to heat an additional aerosol precursor to produce an additional aerosol.

In a ninth aspect, there is provided a smoking substitute device including the aerosol delivery device according to the eighth aspect.

In a tenth aspect, there is provided a method of activating the aerosol delivery device of the eighth aspect, comprising the step of applying a force to the frangible seal to break it.

The method may further comprise a step, performed before or after the step of applying the force, of moving the aerosol generator relative to the reservoir. The step of moving the aerosol generator relative to the reservoir may cause a piercing member to open a pressure relief opening in the reservoir.

In any of the aspects described above, the aerosol delivery device may be a consumable for a vaping device.

In any of the aspects described above, the aerosol delivery device may further comprise an additional aerosol generator, the additional aerosol generator configured to produce an additional aerosol from an additional aerosol precursor.

In any of the aspects described above, the additional aerosol generator may be configured to heat the additional aerosol precursor to form the additional aerosol.

The disclosure includes the combination of the aspects and preferred features described except where such a combination is clearly impermissible or expressly avoided.

### Summary of the Figures

So that the discsloure may be understood, and so that further aspects and features thereof may be appreciated, embodiments illustrating the principles will now be discussed in further detail with reference to the accompanying figures, in which:
**Fig. 1** shows a schematic drawing of a smoking substitute device;
**Fig. 2** shows a schematic drawing of a smoking substitute device;
**Fig. 3** shows a schematic drawing of a smoking substitute device;
**Fig. 4** shows a schematic drawing of a smoking substitute device;
**Fig. 5** shows a cross-sectional view of a consumable in a deactivated state;
**Fig. 6** shows a cross-sectional view of the consumable of Fig. 5 in an activated state;
**Fig. 7** shows a cross-sectional view of a flavour pod portion of a consumable;
**Fig. 8a** shows a top view of a flavour pod portion of a consumable;
**Fig. 8b** shows a cut away perspective view of a flavour pod portion of a consumable;
**Fig. 9** shows a cross-sectional view of a flavour pod portion of a consumable;
**Fig. 10** shows a cross-sectional view of a flavour pod portion of a consumable;
**Fig. 11** shows a cross-sectional view of a flavour pod portion of a consumable;
**Fig. 12** shows a cross-sectional view of the consumable of Figure 11 in an activated state.
**Fig. 13** shows a cross-sectional view of a consumable in a deactivated state;
**Fig. 14** shows a cross-sectional view of a consumable in an activated state;
**Fig. 15** shows a cross-sectional view of a flavour pod portion in a deactivated state; and
**Fig. 16** shows a cross-sectional view of the flavour pod portion of Fig. 15 in an activated state;
**Fig. 17** shows a cut away view of a flavour pod portion of a consumable; and
**Fig. 18** shows a cross-sectional view of a consumable.

### Detailed Description

Aspects and embodiments will now be discussed with reference to the accompanying figures. Further aspects and embodiments will be apparent to those skilled in the art.

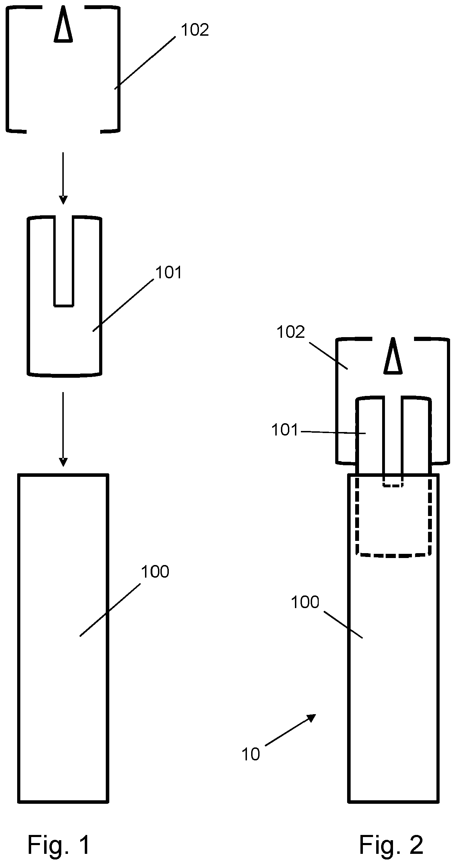

Referring to Figures 1 and 2, there is shown a smoking substitute device 10. In this example, the smoking substitute device comprises a cartomiser 101 and a flavour pod 102 connected to a base unit 100. In this example, the base unit 100 includes elements of the smoking substitute device such as a battery, an electronic controller, and a pressure transducer. The cartomiser 101 may engage with the base unit 100 via a push-fit engagement, a screw-thread engagement, or a bayonet fit, for example. A cartomiser may also be referred to as a "pod". The smoking substitute device can include an aerosol delivery device according to the present disclosure.

The flavour pod 102 is configured to engage with the cartomiser 101 and thus with the base unit 100. The flavour pod 102 may engage with the cartomiser 101 via a push-fit engagement, a screw-thread engagement, or a bayonet fit, for example. Fig. 2 illustrates the cartomiser 101 engaged with the base unit 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device according to the present disclosure.

As will be appreciated from the following description, the cartomiser 101 and the flavour pod 102 may alternatively be combined into a single component that implements the functionality of the cartomiser 101 10 and flavour pod 102. Such a single component may also be an aerosol delivery device according to the present disclosure. In other examples, the cartomiser may be absent, with only a flavour pod 102 present.

A "consumable" component may mean that the component is intended to be used once until exhausted, and then disposed of as waste or returned to a manufacturer for reprocessing.

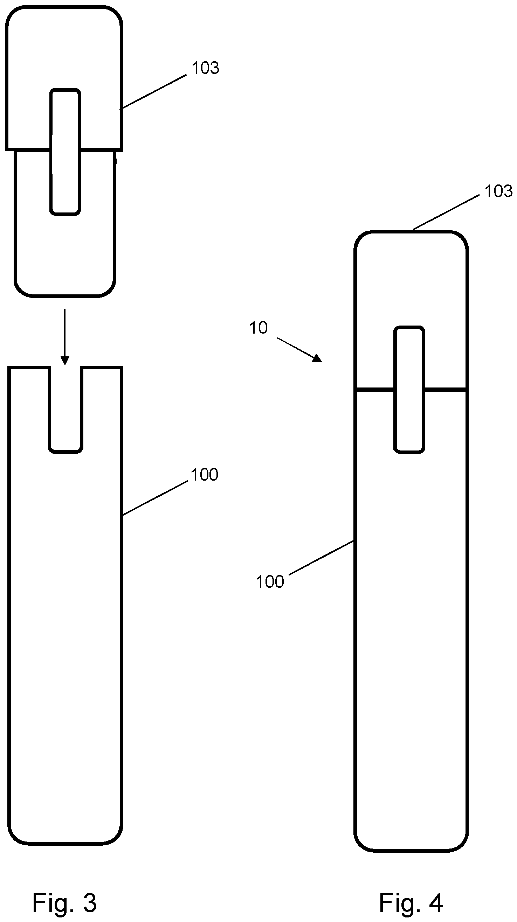

Referring to Figures 3 and 4, there is shown a smoking substitute device comprising a base unit 100 and a consumable 103. The consumable 103 combines the functionality of the cartomiser 101 and the flavour pod 102. In Figure 3, the consumable 103 and the base unit 100 are shown separated from one another. In Figure 4, the consumable 103 and the base unit 100 are engaged with each other to form the smoking substitute device 10.

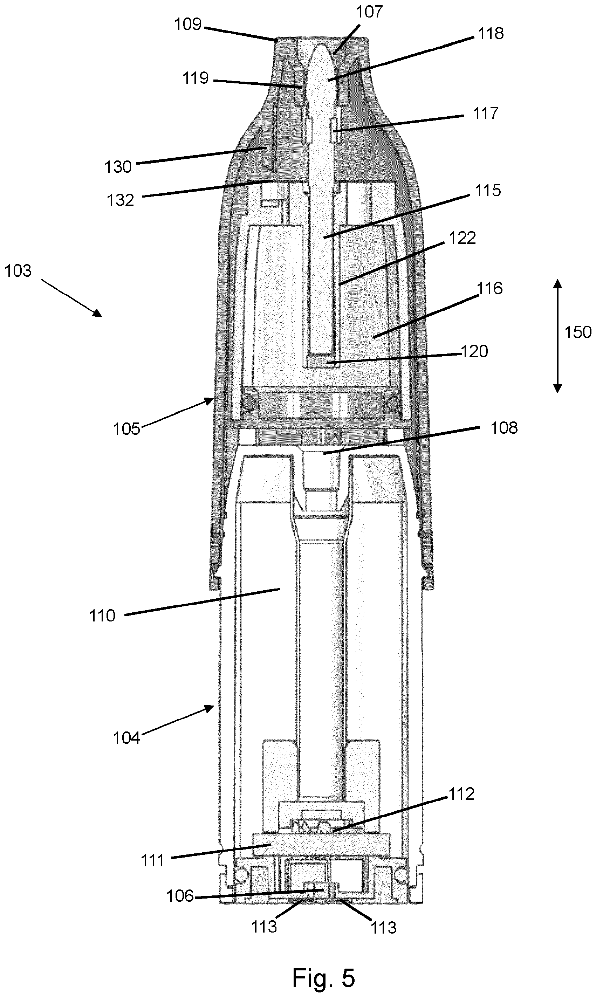

Referring to Figure 5, there is shown a consumable 103 engagable with a base unit via a push-fit engagement in a deactivated state. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a liquid transfer element 115, in the form of a member 115. The member 115 acts as a passive aerosol generator (e.g. an aerosol generator which does not use heat to form the aerosol, also referred to as a "first aerosol generator" in this example), and is formed of a porous material. The member 115 comprises a supporting portion 117, which is located inside a housing, and an aerosol generator portion 118, which is located in the airflow passage 108. In this example, the aerosol generator portion 118 is a porous nib.

When activated, as discussed in more detail below, a first storage 116 (in this example a tank) for storing a first aerosol precursor (i.e. a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage 116 into the member 115 via a wicking action.

Before activation, the barrier arrangement 120 is closed and inhibits evaporation of aerosol precursor. In this example, this is achieved by the barrier arrangement inhibiting flow of aerosol precursor from the first storage 116 to the member 115. In order to inhibit flow of aerosol precursor, the barrier arrangement 120 substantially isolates the first storage 116 from the member 115. In this example, the barrier arrangement comprises a plug 120 (preferably formed from silicon) located at one end of a tube 122 containing the member 115 close to the first storage 116. The tube 122 may be an example of the frangible seal discussed above in relation to the eighth aspect. To activate the consumable 103, a user may squeeze the flavour pod portion 105 thereby braking the frangible seal.

In other examples, the plug may be replaced by a deformable and/or breakable barrier component, e.g. any one of: a duck bill valve; a split valve or diaphragm; or a sheet of foil, which may be pierced by the member 115 when opening the barrier arrangement.

The first storage 116 further includes a pressure relief opening 132, which in the deactivated state is sealed by blocking arrangement. In this example, the blocking arrangement comprises a pierceable cover (preferably made from foil). Piercing member 130, which is formed as a part of the mouthpiece 109 and may take the form of a blade, pierces the pierceable cover and opens the pressure relief opening 132 when the consumable is moved to the activated state (as is discussed in more detail below). This means that opening of the barrier arrangement also effects opening of the blocking arrangement.

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increased air velocity and corresponding reduction in air pressure at the aerosol generator portion 118, the constriction is a Venturi aperture 119. The constriction is generally toroidal in shape, and may include one or more intersections where supports contact the aerosol generator portion 118.

The cartomiser portion 104 of the consumable 103 includes a second storage 110 (in this example a tank) for storing a second aerosol precursor (i.e. e-liquid, which may contain nicotine). Extending into the second storage 110 is a wick 111. The wick 111 is formed from a porous wicking material (e.g. a polymer) that draws second aerosol precursor from the second storage 110 into a central region of the wick 111 that is located outside the e-liquid storage tank 110.

A heater 112 is a configured to heat the central region of the wick 111. The heater 112 includes a resistive heating filament that is coiled around the central region of the wick 111. The wick 111, the heater 112 and the e-liquid storage tank 110 together act as an active aerosol generator (i.e. an aerosol generator which uses heat to form the aerosol, referred to as a "second aerosol generator" in this example).

As described above, the first and second aerosol generators are both at least partially located within the airflow passage 108, with the first aerosol generator downstream (with respect to air flow in use) of the second aerosol generator.

So that the consumable 103 may be supplied with electrical power for activation of the heater 112, the consumable 103 includes a pair of consumable electrical contacts 113. The consumable electrical contacts 113 are configured for electrical connection to a corresponding pair of electrical supply contacts in the base unit 100. The consumable electrical contacts 113 are electrically connected to the electrical supply contacts 114 when the consumable 103 is engaged with the base unit 100. The base unit 100 includes an electrical power source (not shown), for example a battery.

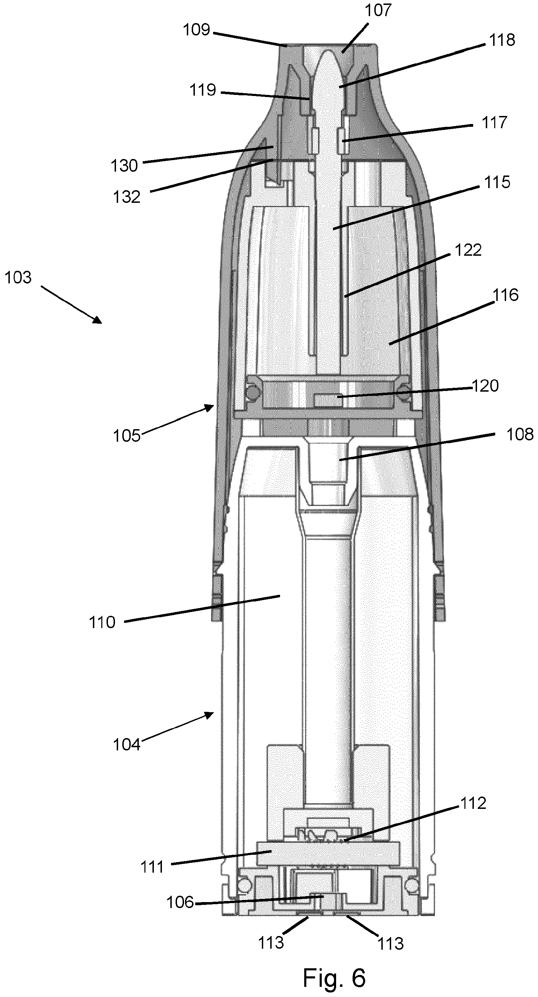

Figure 6 shows the consumable 103 of Figure 5 in an activated state, like features are indicated by like reference numerals. To transition from the deactivated state to the activated state, mouthpiece 109 is moved along a central axis 150 towards cartomizer portion 104 (e.g. one along which the consumable extends, and along which member 115 extends). Moving the mouthpiece 109 in this way effects relative movement between the liquid transfer element (i.e. the member 115) and the barrier arrangement. This causes the barrier arrangement to open. In other examples, a switch is provided for opening the barrier arrangement.

The mouthpiece 109, via supporting portion 117, is fixed to the member 115 and therefore member 115 moves with the mouthpiece 109. The mouthpiece 109, and member 115, is moved relative to the tank 116. This causes displacement of the plug 120 and opening of the barrier arrangement 116.

At the same time, movement of the mouthpiece 109 causes the piercing member 130 to contact and pierce pressure relief opening 132, thereby fluidly connecting the airflow passage 108 to an interior of the first storage 116. This permits air to flow into the first storage 116 as the first storage empties of aerosol precursor in use.

In the present example, once the barrier arrangement is open, the plug 120 is unconstrained within the first storage. However, in other cases, the plug 120 may be received by a guide for inhibiting return of the plug to the closed position after displacement of the plug. The guide may comprise a recess for receiving the plug 120.

In the present example, the barrier arrangement remains permanently open after opening, as the plug 120 does not return to the tube 122. However, in other examples, the barrier arrangement is selectively openable and closable by the user. This may be achieved by the plug (or another type of barrier arrangement) being fixed to an end portion of the member 115, such that the member 115 is selectively exposable to the first storage 116.

Once activated, and in use, a user draws (or "sucks", "pulls", or "puffs") on the mouthpiece 109 of the consumable 103, which causes a drop in air pressure at the outlet 107, thereby generating air flow through the inlet 106, along the airflow passage 108, out of the outlet 107 and into the user's mouth.

When the heater 112 is activated (by passing an electric current through the heating filament in response to the user drawing on the mouthpiece 109, the drawing of air may be detected by a pressure transducer) the e-liquid located in the wick 111 adjacent to the heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The base unit 100 supplies electrical current to the consumable electrical contacts 113. This causes an electric current flow through the heating filament of the heater 112 and the heating filament heats up. As described, the heating of the heating filament causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure and high air velocity region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol is sized to inhibit pulmonary penetration. The first aerosol is formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol is sized for transmission within at least one of a mammalian oral cavity and a mammalian nasal cavity. The first aerosol is formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

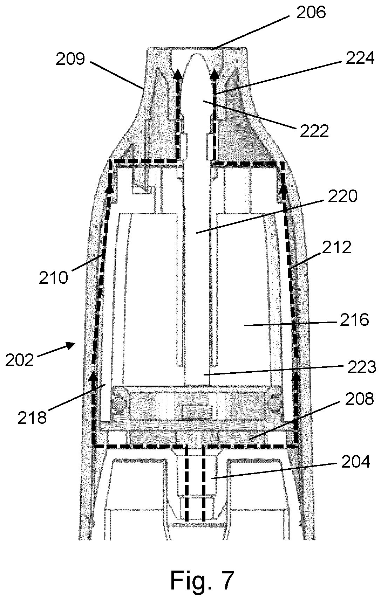

Referring to Fig. 7, there is shown a flavour pod portion 202 of a consumable in an activated state, the consumable providing an aerosol delivery device. The consumable further comprises a cartomiser portion (not shown in Fig. 7) having all of the features of the cartomiser portion 104 described above with respect to Figs. 5 and 6. However, in other examples, the consumable does not comprise the cartomiser portion, and provides only flavour to the user.

The flavour pod portion 202 comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly connecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage, which stores a first aerosol precursor. The storage comprises a reservoir 216 located within a chamber 218. The reservoir 216 is formed of a first porous material.

The flavour pod portion 202 comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the reservoir 216. The member 220 is in contact with the reservoir 216. More specifically, the supporting portion 223 extends into and 6 is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

In use, when a user draws on the mouthpiece 209, air flow is generated through the air flow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

As the first aerosol precursor is picked up by the air, the member 220 transfers further first aerosol precursor from the reservoir 216 to the aerosol generator portion 222. More specifically, the member 220 wicks the first aerosol precursor from the reservoir 216 to the aerosol generator portion 224.

In other examples, the storage comprises a tank containing the first aerosol precursor as free liquid, rather than the reservoir 216 and the chamber 218. In such examples, the member 220 still extends into the tank to transfer first aerosol precursor from the tank to the aerosol generator portion 224.

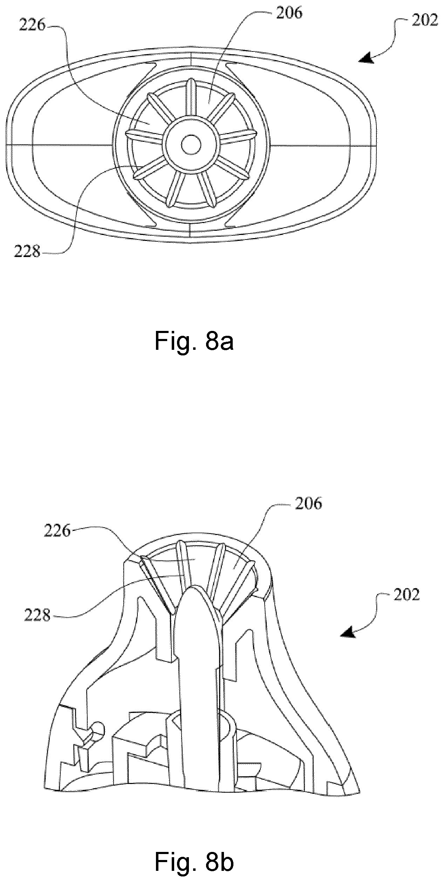

Figs. 8a and 8b show further views of the flavour pod portion 202 which highlight features of the mouthpiece 209. Many of the reference numerals of Fig. 7 are omitted from Fig. 8a and 8b for clarity.

The mouthpiece aperture 206 comprises an inner surface 226, which is uneven. In the present example, the inner surface 226 has the form of a substantially frustoconical surface, but includes grooves or channels 228 to make the inner surface 226 somewhat uneven. In other examples, the inner surface 226 may have another form (for example, the form a substantially cylindrical surface), and may include any type of protrusion or groove to make the inner surface uneven.

The inner surface 226 is angled with respect to an axial direction (i.e. relative to a central axis extending from a base of the consumable to the mouthpiece) such that the width of the mouthpiece aperture 209 increases in the downstream direction. The inner surface 226 is immediately downstream of the narrowing section 224 of the airflow passage 108.

The grooves 228 are generally v-shaped in cross-sectional profile, and extend in the axial direction for the full length of the inner surface 226. Each groove 228 is formed from a pair of surfaces angled at between 30 and 90 degrees relative to each other. More specifically, each groove 228 is formed from a pair of surfaces angled at 60 degrees relative to each other.

The grooves 228 have a depth (measured normal to the inner surface 226) of at least 0.2 mm. More specifically, the grooves 228 have a depth of at least 0.3 mm. More specifically, the grooves 228 have a depth of at least 0.4 mm.

The grooves 228 have a depth of less than 0.8 mm. More specifically, the grooves have a depth of less than 0.7 mm. More specifically, the grooves have a depth of less than 0.6 mm.

More specifically, the grooves have a depth of substantially 0.5 mm.

The grooves 228 are substantially equi-spaced in a circumferential manner around the inner surface 226. The inner surface 226 comprises at least 6 grooves. More specifically, the inner surface comprises at least 7 grooves. More specifically, the inner surface 226 comprises at least 8 grooves.

The inner surface 226 comprises at most 12 grooves 228. More specifically, the inner surface 226 comprises at most 11 grooves 228. More specifically, the inner surface 226 comprises at most 10 grooves 228.

More specifically, the inner surface 226 comprises 9 grooves 228.

The grooves 228 are spaced apart from each other by substantially 1 mm at the downstream end of the inner surface 226. In other examples, the spacing at the downstream end of grooves or protrusions may be selected such that it is equal to or less than the mass median diameter (as described above) of particles in the first aerosol.

The inner surface 226 comprises a smooth polished surface between the grooves 228. Polishing the surface in this way provides improved aerodynamic properties. However, in other examples, the inner surface 226 may be textured. In such examples, the texture of the surface may provide the uneven surface, and no grooves are required,

In use, the uneven nature of the inner surface 226 makes it easier for droplets to form on the inner surface 226, preventing large droplets from entering the user's mouth. The grooves 228 help to channel the large droplets back into the consumable.

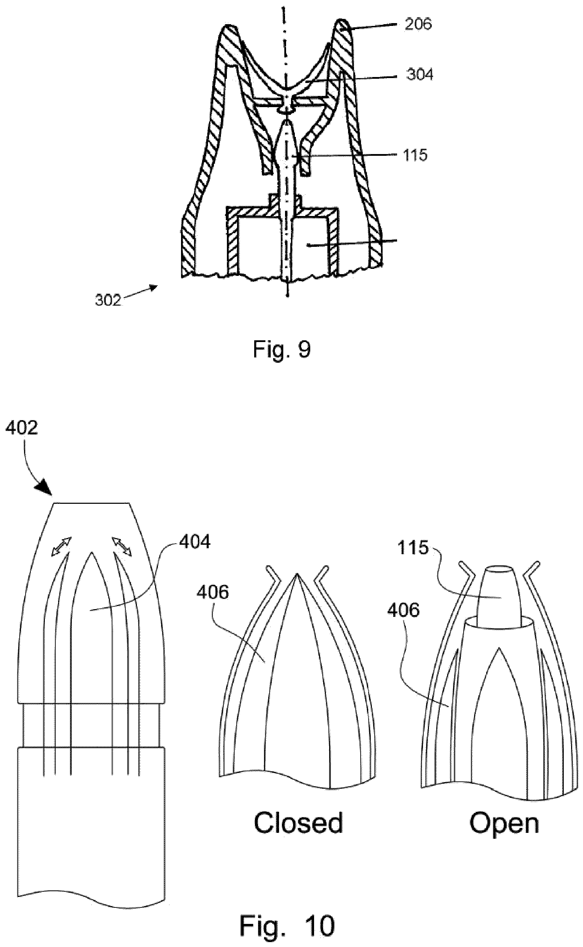

Referring to Fig. 9 there is shown a sectional drawing of a second flavour pod portion 302. The second flavour pod portion 302 comprises all of the features of the flavour pod portion 202 aside from the differences described here. Many of the reference numerals relating to features which are common between the second flavour pod portion 302 and the flavour pod portion 202 are omitted from Fig. 9 for clarity. However, like reference numerals are used in Fig. 9 where features referred to previously are referred to again.

The second flavour pod portion 302 comprises a second barrier arrangement 304 for inhibiting evaporation of aerosol precursor when the second barrier arrangement 304 is closed. The second barrier arrangement 302 does not inhibit flow between the first storage and the member 115, which means that the member 115 contains aerosol precursor even when the second barrier arrangement 304 is closed. The second barrier arrangement 304 comprises a valve 304 which is located in the outlet 206 of the second flavour pod portion 302. The valve 304 is a diaphragm valve. The valve 304 substantially seals the outlet 206.

In use, the user inhales on the mouthpiece, which causes the second barrier arrangement 302/valve 304 to open, permitting aerosol to flow out of the outlet 206. The second barrier arrangement 302 closes when the user stops inhaling.

Referring to Fig. 10 there is shown a sectional drawing of a third flavour pod portion 402. The third flavour pod portion 402 comprises all of the features of the flavour pod portion 202 aside from the differences described here. Many of the reference numerals relating to features which are common between the second flavour pod portion 402 and the flavour pod portion 202 are omitted from Fig. 10 for clarity. However, like reference numerals are used in Fig. 10 where features referred to previously are referred to again.

The third flavour pod portion 402 comprises a third barrier arrangement 404 for inhibiting evaporation of aerosol precursor when the third barrier arrangement 404 is closed. As with the second barrier arrangement 302, the third barrier arrangement 402 does not inhibit flow between the first storage and the member 115, which means that the member 115 contains aerosol precursor even when the third barrier arrangement 404 is closed. The third barrier arrangement 404 is a shield 404. The shield 404 is formed of a plastic material. The shield 404 encloses the member 115 when the shield 404 is closed.

The shield 404 comprises four curved plates 406. When the shield 404 opens, the curved plates 406 separate and slide to expose the member 115 to permit generation of aerosol.

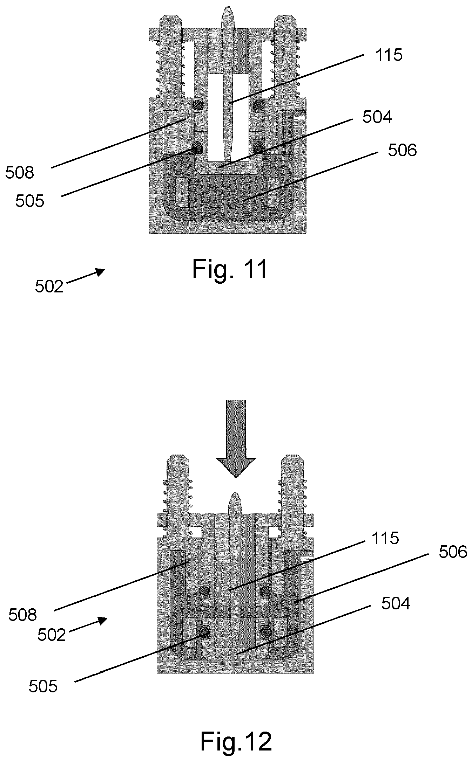

Referring to Fig. 11 and Fig. 12 there are shown cross sectional drawings of a fourth flavour pod portion 502 in deactivated and activated states respectively. The fourth flavour pod portion 502 comprises all of the features of the flavour pod portion 202 aside from the differences described here. Many features and reference numerals relating to features which are common between the fourth flavour pod portion 502 and the flavour pod portion 202 are omitted from Fig. 11 and 12 for clarity. However, like reference numerals are used in Fig. 11 and 12 where features referred to previously are referred to again.

The fourth flavour pod portion 502 comprises a fourth barrier arrangement 504 and a second storage 506. The fourth barrier arrangement 504 is configured to inhibit the flow of aerosol precursor from the storage 506 to the transfer element 115 (i.e. the member 115) when the barrier arrangement is in a closed position, as shown in Fig. 11. The fourth barrier arrangement 504 comprises a barrier element 504. The barrier element 504 is fixed to an end portion of the member 115. The barrier element 504 encloses the end portion of the member 115.

The member 115 and the barrier element 504 are located in a tube 508. The barrier element 504 blocks the tube 508 to prevent flow of aerosol precursor to the tube 508 when the fourth barrier arrangement 504 is in the closed position. The barrier element 504 comprises an o-ring 505, which extends around the member 115 to seal the tube 508.

In order to open the fourth barrier arrangement 504, the user slides the mouthpiece (not shown) to effect sliding of the member 115 and the barrier element 504. This causes the barrier element 504 and a side portion 310 of the member 115 to leave the tube 508, thereby exposing the side portion 310 to the aerosol precursor in the storage 506. Aerosol precursor then moves from the storage 506 and into the member for aerosolisation. Fig. 12 shows the fourth barrier arrangement 504 in the open position.

The fourth barrier arrangement 504 can then be closed by the user by sliding the mouthpiece (or in other examples a switch) in the opposite direction. This returns the member 115 and the barrier element 504 to the tube 508 such that the barrier element 504 seals the tube 508 again. In this way, the fourth barrier arrangement 504 is selectively openable and closable by the user. If the fourth barrier arrangement 504 is closed again, aerosol will be produced for a small number of "puffs" before the aerosol precursor in the member 115 is depleted. The user can then choose to open the fourth barrier arrangement 504 if required.

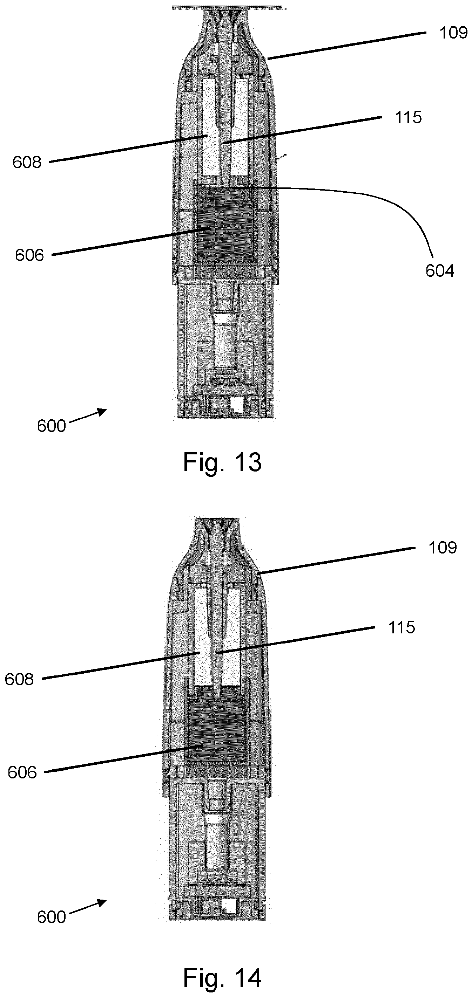

Referring to Fig. 13 and Fig. 14 there are shown cross sectional drawings of a further consumable 600 comprising a fifth flavour pod portion 602 in activated and deactivated states respectively. The fifth flavour pod portion 602 comprises all of the features of the flavour pod portion 202 aside from the differences described here. Many of the reference numerals relating to features which are common between the fifth flavour pod portion 602 and the flavour pod portion 202 are omitted from Fig. 13 for clarity. However, like reference numerals are used in Fig. 13 where features referred to previously are referred to again.

The fifth flavour pod portion 602 comprises a storage 606 and an additional storage 608. The storage 606 is a free liquid tank and the additional storage 608 is a porous reservoir. Aerosol precursor is initially stored in the storage 606 with the additional storage 608 empty.

The fifth flavour pod portion 602 comprises a fifth barrier arrangement 604 for inhibiting evaporation of aerosol precursor when the fifth barrier arrangement 604 is closed. The fifth barrier arrangement 604 inhibits evaporation of aerosol precursor by preventing flow of aerosol precursor between the storage 606 and the additional storage 608. As can be seen in Fig. 13, when the fifth barrier arrangement is closed, the member 115 is located within and in contact with the empty additional storage 608. The member 115 is distinct from the storage 606.

When the fifth barrier arrangement is closed, the member 115 is not in contact with the storage 606. The fifth barrier arrangement 604 comprises a pierceable substrate (in this example a foil substrate) located between the storage 606 and the additional storage 608.

As before, to open the fifth barrier arrangement 604, the mouthpiece 109 is moved relative to a body of the further consumable 600, which effects relative movement between the liquid transfer element (i.e. the member 115) and the fifth barrier arrangement 604. The movement of the member 115 causes the member to pierce the substrate of the fifth barrier arrangement 604, thereby opening the fifth barrier arrangement 604. This allows aerosol precursor to flow from the storage 606 to the additional storage 608 and into the member 115 for aerosolisation.

The user may need to turn the further consumable 600 upside down in order to move aerosol precursor to the additional storage 608. Aerosol precursor is then held in the porous reservoir of the additional storage 608. The fifth barrier arrangement 604 remains permanently open after opening.

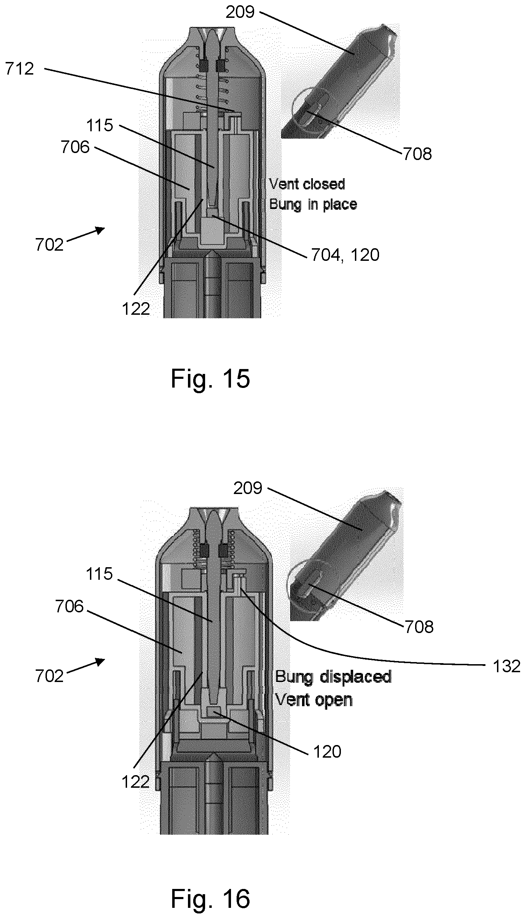

Referring to Fig. 15 and 16 there is shown a sectional drawing of a sixth flavour pod portion 702 in a deactivated and activated state respectively. The sixth flavour pod portion 702 comprises all of the features of the flavour pod portion 202 aside from the differences described here. Many of the reference numerals relating to features which are common between the sixth flavour pod portion 702 and the flavour pod portion 202 are omitted from Fig. 15 for clarity. However, like reference numerals are used in Fig. 15 where features referred to previously are referred to again.

The sixth flavour pod portion 702 comprises a sixth barrier arrangement 704 and a storage 706. As with the first barrier arrangement, the sixth barrier arrangement 704 comprises a plug 120 located at one of the tube 122 when the sixth barrier arrangement 704 is in the closed position as shown in Fig. 15. However, unlike the first barrier arrangement, the sixth barrier arrangement 704 is openable by moving the storage 706 with respect to the sixth barrier arrangement 704. More specifically, the sixth barrier arrangement 704 is openable by sliding the storage 706 with respect to the sixth barrier arrangement 704.

Since the tube 122 is integrally formed with the storage 706, moving the storage 706 in this way causes relative movement between the storage 706 and the member 115, causing the member 115 to displace the plug 120 from the tube 122 to open the sixth barrier arrangement 704. The member 115 is held in position relative to the mouthpiece by the second supporting portion 710.

The sixth flavour pod portion 702 comprises a switch 708. The switch 708 is located on a front face of the sixth flavour pod portion 702. As shown in Fig. 15 and Fig. 16, the switch 708 is slidable. Sliding of the switch 708 effects sliding of the storage 706 to open the sixth barrier arrangement 704.

The sixth flavour pod portion 702 comprises a blocking arrangement 712. The blocking arrangement 712 inhibits flow through the pressure relief opening 132 when the blocking arrangement 712 is in the closed position as in Fig. 15.

When the sixth barrier arrangement 704 is in the closed position as in Fig. 15, the blocking arrangement 712 is connected to the switch 708 and the storage 706. This means that sliding of the switch 708 causes sliding of the blocking arrangement 712, which in turn causes sliding of the storage 706.

The sixth flavour pod portion 702 comprises a stop (not shown). The stop prevents the storage moving beyond a point. Beyond this point, further moving of the switch 708 causes the blocking arrangement 712 to disconnect from the storage 706. This causes the blocking arrangement 712 to move away from the pressure relief opening 732 to open the blocking arrangement 712.

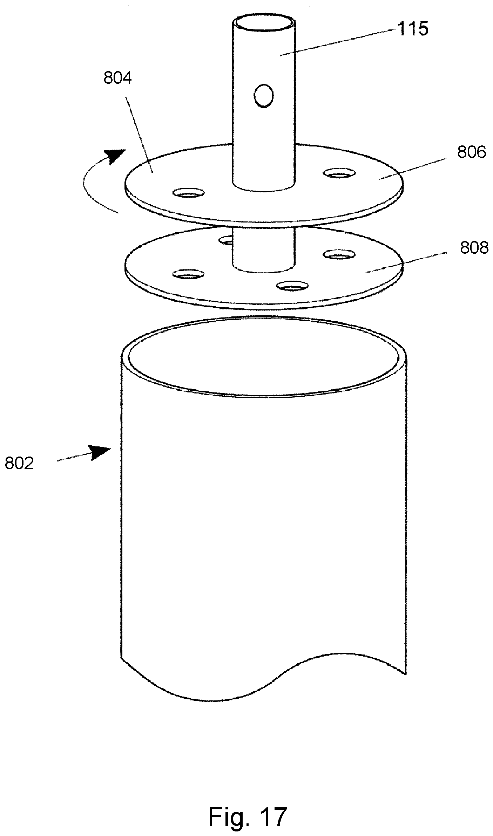

Referring to Fig. 17 there is shown a sectional drawing of a seventh flavour pod portion 802. The seventh flavour pod portion 802 comprises all of the features of the flavour pod portion 202 aside from the differences described here. Many of the reference numerals relating to features which are common between the seventh flavour pod portion 802 and the flavour pod portion 202 are omitted from Fig. 17 for clarity. However, like reference numerals are used in Fig. 17 where features referred to previously are referred to again.

The seventh flavour pod portion 802 does not comprise a barrier arrangement as described previously, including only a second blocking arrangement 804. The second blocking arrangement also inhibits evaporation from the aerosol generator portion (by preventing air flow into the storage), in some cases to a sufficient extent.

The second blocking arrangement 804 comprises first and second blocking plates 806, 808. The first and second blocking plates 806, 808 are rotatable relative to each other to selectively line up apertures in the first and second blocking plates 806, 808. When the apertures in the first and second blocking plates 806, 808 are lined up, a pressure relief valve (i.e. permitting air flow into the storage) is opened. The first blocking plate 806 is fixed to the mouthpiece. In this way, rotation of the mouthpiece relative to the body of the device permits selective opening of the second blocking arrangement 804.

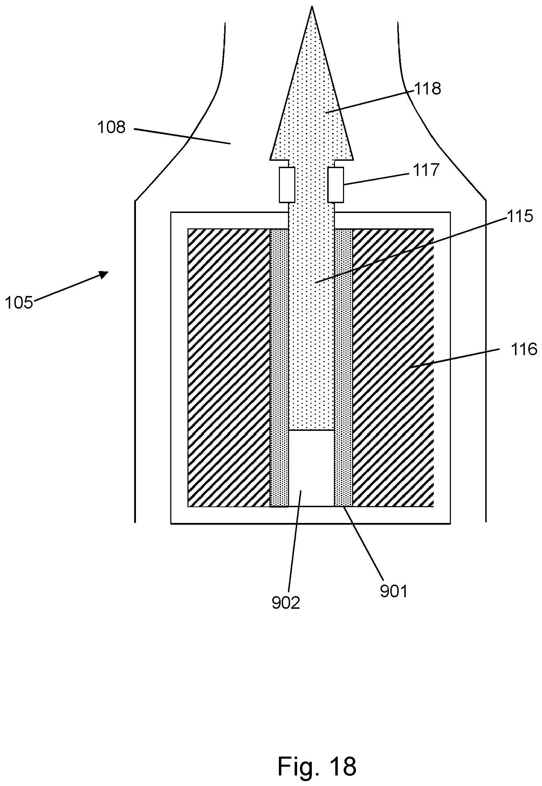

Figure 18 shows a cross-sectional view of a flavour pod portion 105 of a consumable. As before, member 115 functions as a passive aerosol generator and is formed of a porous material. The member includes an aerosol generator portion 118, located within airflow passage 108, and is supported via supporting portion 117. The member 115 is partially located within the first storage 116 which stores a first aerosol precursor. In the state shown in Figure 18, the member is fluidly isolated from the first aerosol precursor by frangible seal 901. In this example, the frangible seal 901 is a cylindrical glass tube which seals member 115 from the contents of the first storage 116.

In use, the user applies a force to the first storage 116 (for example, by squeezing the casing of the flavour pod 105) which in turn applies a force to the frangible seal 901. Advantageously, the first storage 116 containing first aerosol precursor is, before activation, substantially full of first aerosol precursor such that the squeezing force is efficiently transferred to the frangible seal 901 (the first aerosol precursor being, in this example, an essentially incompressible liquid).

Moreover, there is, in this example, a void 902 containing air or another gas and notably not containing any portion of the member 115. Advantageously, this can help ensure that the frangible seal breaks with less application than if the member 115 extended all of the way to a base of the first storage 116 i.e. the user does not need to apply a force sufficient to compress the member 115, but rather just compress the gas within void 902. However in other examples (not shown) the member 115 extends to the base of the first storage 116 such that there is no void 902 present. The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined in the appended claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

## Claims

1. An aerosol delivery device comprising:
a storage (116) for storing an aerosol precursor;
an aerosol generator (115) comprising an aerosol generator portion (118) configured to receive the aerosol precursor from the storage, the aerosol generator being a passive aerosol generator such that the air directed past the aerosol generator portion picks up the aerosol precursor from the aerosol generator portion to form an aerosol;
an air flow passage (208) configured to direct air past the aerosol generator portion;
**characterized in that**:
the device further comprises a barrier arrangement (120, 304, 404, 504, 604, 704) for inhibiting evaporation of aerosol precursor when the barrier arrangement is closed, the barrier arrangement being openable to permit generation of aerosol, wherein the barrier arrangement (504) comprises a barrier element between the aerosol generator portion and a mouthpiece aperture of the aerosol delivery device to inhibit evaporation of aerosol precursor.

2. An aerosol delivery device according to claim 1, wherein the barrier arrangement (120, 304, 404, 504, 604, 704) is configured to open in response to a user inhaling on the aerosol delivery device.

3. An aerosol delivery device according to claim 1 or claim 2, wherein the barrier arrangement (504) is selectively openable by a user.

4. An aerosol delivery device according to claim 3, and further comprising a mouthpiece and a body, wherein the barrier arrangement (504) is configured to open in response to sliding of the mouthpiece relative to the body.

5. An aerosol delivery device according to any one of claims 1 to 4, wherein the barrier element comprises a non-return valve.

6. An aerosol delivery device according to any one of claims 1 to 4, wherein the barrier element (404) comprises a movable shield.

7. An aerosol delivery device according to any one of the preceding claims, further comprising:
a pressure relief opening (132) in the storage (116); and
a blocking arrangement (712) for inhibiting flow through the pressure relief opening,
wherein the blocking arrangement is openable to permit air to flow through the pressure relief opening and into the storage as the storage empties of aerosol precursor.

8. An aerosol delivery device according to any one of the preceding claims, wherein the aerosol delivery device is a consumable for a vaping device.

9. An aerosol delivery device according to any one of the preceding claims and further comprising an additional aerosol generator (110, 111, 112), the additional aerosol generator configured to produce an additional aerosol from an additional aerosol precursor.

10. An aerosol delivery device according to claim 9, wherein the additional aerosol generator (110, 111, 112) is configured to heat the additional aerosol precursor to form the additional aerosol.

## Patentansprüche

1. Aerosolabgabevorrichtung, umfassend:
einen Speicher (116) zum Speichern eines Aerosolvorläufers;
einen Aerosolgenerator (115), umfassend einen Aerosolgeneratorabschnitt (118), der dazu ausgelegt ist, den Aerosolvorläufer aus dem Speicher aufzunehmen, wobei der Aerosolgenerator ein passiver Aerosolgenerator ist, sodass die am Aerosolgeneratorabschnitt vorbeigeleitete Luft den Aerosolvorläufer aus dem Aerosolgeneratorabschnitt mitreißt, um ein Aerosol zu bilden;
einen Luftströmungsdurchgang (208), der dazu ausgelegt ist, Luft am Aerosolgeneratorabschnitt vorbeizuleiten;
**dadurch gekennzeichnet, dass**:
die Vorrichtung ferner eine Barriereanordnung (120, 304, 404, 504, 604, 704) umfasst, um ein Verdampfen des Aerosolvorläufers zu hemmen, wenn die Barriereanordnung geschlossen ist, wobei die Barriereanordnung öffenbar ist, um das Erzeugen von Aerosol zu ermöglichen, wobei die Barriereanordnung (504) ein Barriereelement zwischen dem Aerosolgeneratorabschnitt und einer Mundstücköffnung der Aerosolabgabevorrichtung umfasst, um ein Verdampfen des Aerosolvorläufers zu hemmen.

2. Aerosolabgabevorrichtung nach Anspruch 1, wobei die Barriereanordnung (120, 304, 404, 504, 604, 704) dazu ausgelegt ist, sich in Reaktion auf das Inhalieren an der Aerosolabgabevorrichtung durch einen Benutzer zu öffnen.

3. Aerosolabgabevorrichtung nach Anspruch 1 oder 2, wobei die Barriereanordnung (504) durch einen Benutzer selektiv öffenbar ist.

4. Aerosolabgabevorrichtung nach Anspruch 3 und ferner umfassend ein Mundstück und einen Körper, wobei die Barriereanordnung (504) dazu ausgelegt ist, sich in Reaktion auf ein Gleiten des Mundstücks relativ zum Körper zu öffnen.

5. Aerosolabgabevorrichtung nach einem der Ansprüche 1 bis 4, wobei das Barriereelement ein Rückschlagventil umfasst.

6. Aerosolabgabevorrichtung nach einem der Ansprüche 1 bis 4, wobei das Barriereelement (404) eine bewegbare Abdeckung umfasst.

7. Aerosolabgabevorrichtung nach einem der vorangegangenen Ansprüche, ferner umfassend:
eine Druckentlastungsöffnung (132) im Speicher (116); und
eine Sperranordnung (712) zum Hemmen von Strömung durch die Druckentlastungsöffnung,
wobei die Sperranordnung öffenbar ist, um das Strömen von Luft durch die Druckentlastungsöffnung und in den Speicher zu ermöglichen, wenn der Speicher sich hinsichtlich des Aerosolvorläufers entleert.

8. Aerosolabgabevorrichtung nach einem der vorangegangenen Ansprüche, wobei die Aerosolabgabevorrichtung eine Verbrauchsware für eine Vaping-Vorrichtung ist.

9. Aerosolabgabevorrichtung nach einem der vorangegangenen Ansprüche und ferner umfassend einen zusätzlichen Aerosolgenerator (110, 111, 112), wobei der zusätzliche Aerosolgenerator dazu ausgelegt ist, ein zusätzliches Aerosol aus einem zusätzlichen Aerosolvorläufer zu erzeugen.

10. Aerosolabgabevorrichtung nach Anspruch 9, wobei der zusätzliche Aerosolgenerator (110, 111, 112) dazu ausgelegt ist, den zusätzlichen Aerosolvorläuferzu erhitzen, um das zusätzliche Aerosol zu bilden.

## Revendications

1. Dispositif de distribution d'aérosol, comprenant :
un dispositif de stockage (116) pour stocker un précurseur d'aérosol ;
un générateur d'aérosol (115) comprenant une partie de générateur d'aérosol (118) configurée pour recevoir le précurseur d'aérosol provenant du dispositif de stockage, le générateur d'aérosol étant un générateur d'aérosol passif de telle sorte que l'air dirigé au-delà de la partie de générateur d'aérosol recueille le précurseur d'aérosol provenant de la partie de générateur d'aérosol pour former un aérosol ;
un passage d'écoulement d'air (208) configuré pour diriger de l'air au-delà de la partie de générateur d'aérosol ;
**caractérisé en ce que** :
le dispositif comprend en outre un agencement de barrière (120, 304, 404, 504, 604, 704) pour empêcher l'évaporation du précurseur d'aérosol lorsque l'agencement de barrière est fermé, l'agencement de barrière pouvant être ouvert pour permettre la génération d'un aérosol, dans lequel l'agencement de barrière (504) comprend un élément de barrière entre la partie de générateur d'aérosol et une ouverture d'embout buccal du dispositif de distribution d'aérosol afin d'empêcher l'évaporation du précurseur d'aérosol.

2. Dispositif de distribution d'aérosol selon la revendication 1, dans lequel l'agencement de barrière (120, 304, 404, 504, 604, 704) est configuré pour s'ouvrir en réponse à l'inhalation d'un utilisateur sur le dispositif de distribution d'aérosol.

3. Dispositif de distribution d'aérosol selon la revendication 1 ou la revendication 2, dans lequel l'agencement de barrière (504) peut être ouvert de manière sélective par un utilisateur.

4. Dispositif de distribution d'aérosol selon la revendication 3, et comprenant en outre un embout buccal et un corps, dans lequel l'agencement de barrière (504) est configuré pour s'ouvrir en réponse au coulissement de l'embout buccal par rapport au corps.

5. Dispositif de distribution d'aérosol selon l'une quelconque des revendications 1 à 4, dans lequel l'élément de barrière comprend un clapet anti-retour.

6. Dispositif de distribution d'aérosol selon l'une quelconque des revendications 1 à 4, dans lequel l'élément de barrière (404) comprend un bouclier mobile.

7. Dispositif de distribution d'aérosol selon l'une quelconque des revendications précédentes, comprenant en outre :
une ouverture de décharge de pression (132) dans le dispositif de stockage (116) ; et
un agencement de blocage (712) pour empêcher un écoulement à travers l'ouverture de décharge de pression,
dans lequel l'agencement de blocage peut être ouvert afin de permettre à de l'air de s'écouler à travers l'ouverture de décharge de pression et jusque dans le dispositif de stockage lorsque le dispositif de stockage se vide de précurseur d'aérosol.

8. Dispositif de distribution d'aérosol selon l'une quelconque des revendications précédentes, dans lequel le dispositif de distribution d'aérosol est un produit consommable pour un dispositif de vapotage.

9. Dispositif de distribution d'aérosol selon l'une quelconque des revendications précédentes et comprenant en outre un générateur d'aérosol supplémentaire (110, 111, 112), le générateur d'aérosol supplémentaire étant configuré pour produire un aérosol supplémentaire à partir d'un précurseur d'aérosol supplémentaire.

10. Dispositif de distribution d'aérosol selon la revendication 9, dans lequel le générateur d'aérosol supplémentaire (110, 111, 112) est configuré pour chauffer le précurseur d'aérosol supplémentaire afin de former l'aérosol supplémentaire.
